# EUROPÄISCHE PATENTANMELDUNG

(11) **EP 3 666 421 A1**
(43) Veröffentlichungstag der Anmeldung: **17.06.2020**
(21) Anmeldenummer: 18211714.3
(22) Anmeldetag: 11.12.2018
(51) Int. Cl.: B22F 3/00, A61B 6/00, B22F 3/105, B22F 3/11, B22F 5/00, B33Y 80/00, B33Y 70/00, B33Y 10/00

(54) **VERFAHREN ZUR HERSTELLUNG EINES DREIDIMENSIONALEN BAUTEILS AUS EINEM METALLSCHAUM**

(71) Anmelder: Siemens Healthcare GmbH, 91052 Erlangen (DE)
(72) Erfinder: Keil, David-Amadeus, 96047 Bamberg (DE); Kuth, Sonja, 91054 Erlangen (DE); Russ, Jochen, 91350 Gremsdorf (DE)

(57) **Zusammenfassung**

Verfahren zur Herstellung eines dreidimensionalen Bauteils (11) aus einem Metallschaum, mit folgenden Schritten:
e) Verwendung eines Schlickers (7) enthaltend ein Metallpulver, ein Treibmittel und ein Lösungsmittel,
f) Aufbringen des Schlickers (7) mittels einer Düsenanordnung (4) in Form einer Schicht auf eine Trägerfläche (3), wobei die Düsenanordnung (4) und die Trägerfläche (3) relativ zueinander bewegt werden,
g) Erwärmen des aufgebrachten Schlickers (7) mittels eines Heizmittels (5) bis zum Aufschmelzen des Metallpulvers, so dass die Metallschmelze durch ein von dem Treibmittel freigesetztes Gas aufschäumt,
h) Abkühlung und Verfestigung der aufgeschäumten Schicht (10),
wobei die Schritte b) - d) beliebig oft wiederholt werden, wobei der Schlicker (7) jeweils auf eine zuvor erzeugte aufgeschäumte Schicht (10) aufgebracht wird.

## Beschreibung

Die Erfindung betrifft ein Verfahren zur Herstellung eines dreidimensionalen Bauteils aus einem Metallschaum.

Der ideale Werkstoff in industriellen Anwendungen, insbesondere im Leichtbau, besitzt eine Kombination aus hoher Festigkeit und geringer Dichte. Realisiert werden kann diese Anforderung durch Verwendung von Metallschäumen, beispielsweise auf Basis von Aluminium oder Aluminiumlegierungen, alternativ sind auch Metallschäume aus Eisen beziehungsweise Stahl im Einsatz. Eine Möglichkeit zur Herstellung eines solchen Metallschaums zur Bildung einer dreidimensionalen Struktur stellt das Einblasen eines Gases, beispielsweise Luft, in eine Metallschmelze dar. Hierbei ist eine Form mit einem Formhohlraum erforderlich, um den Schaum in die gewünschte dreidimensionale Form beziehungsweise Struktur zu bringen.

Ein anderes Verfahren sieht das Einbringen eines Treibmittels, üblicherweise ein Metallhydrid wie beispielsweise Titandihydrid, in eine Metallschmelze und das darauf folgende Aufschäumen des Metalls vor. Der Grund für das Aufschäumen bei Verwendung eines Metallhydrids ist das Freiwerden von Wasserstoff bei Erreichen des Schmelzpunkts des Metallpulvers. Dieses Aufschäumen erfolgt in einer Form, wobei nach dem Erkalten der Schaubblock der Form entnommen werden und anschließend prozessiert werden kann, wobei der geschäumte Block üblicherweise geschnitten wird.

Eine weitere Möglichkeit sieht vor, ein Pulvergemisch aus einem Metallpulver und einem Treibmittelpulver herzustellen, dieses zu einem Grünling zu pressen und anschließend in einem Sinterprozess zu schäumen, wobei auch hier die Sintertemperatur so hoch liegt, dass das Metallpulver schmilzt und es hierbei zum Aufschäumen der Metallschmelze durch den freigesetzten Wasserstoff kommt.

Als eine weitere Verfahrensvariante ist das sogenannte Schlickerreaktionsschaumsinter-Verfahren (SRSS-Verfahren) bekannt. Bei diesem Verfahren wird ein Schlicker hergestellt, indem ein Metallpulver und zumeist pulvriges Treibmittel mit einem Lösungsmittel und einer Säure vermischt werden. Beim Mischen mit der Säure schäumt der Schlicker und kann dann in eine Form gegeben und getrocknet werden. Ein nachfolgender Sinterprozess der ausgehärteten, getrockneten Grünlinge härtet sodann die Struktur.

All diesen Verfahren gemein ist jedoch, dass sich komplexere dreidimensionale Bauteile und Bauteile in kleinen Stückzahlen hierüber nicht ausbilden lassen, da allen Verfahren die Verwendung einer entsprechenden Form zueigen ist und sich bekanntlich entsprechende Press-, Sinter- oder Gießformen nicht beliebig komplex herstellen lassen.

Der Erfindung liegt damit das Problem zugrunde, ein demgegenüber verbessertes Verfahren zur Herstellung eines dreidimensionalen Bauteils aus einem Metallschaum anzugeben.

Zur Lösung dieses Problems ist erfindungsgemäß ein Verfahren mit folgenden Schritten vorgesehen:
a) Verwendung eines Schlickers enthaltend ein Metallpulver, ein Treibmittel und ein Lösungsmittel,
b) Aufbringen des Schlickers mittels einer Düsenanordnung in Form einer Schicht auf eine Trägerfläche, wobei die Düsenanordnung und die Trägerfläche relativ zueinander bewegt werden,
c) Erwärmen des aufgebrachten Schlickers mittels eines Heizmittels bis zum Aufschmelzen des Metallpulvers, so dass die Metallschmelze durch ein von dem Treibmittel freigesetztes Gas aufschäumt,
d) Abkühlung und Verfestigung der aufgeschäumten Schicht, wobei die Schritte b) - d) beliebig oft wiederholt werden, wobei der Schlicker jeweils auf eine zuvor erzeugte aufgeschäumte Schicht aufgebracht wird.

Das erfindungsgemäße additive, auftragende Verfahren sieht ebenfalls die Verwendung eines Schlickers als Basismaterial vor, wobei der Schlicker ein Metallpulver sowie ein Treibmittel, üblicherweise ebenfalls in Pulverform, enthält, die in einer Trägersubstanz, also einem Fluid respektive Lösungsmittel, suspensiert sind. Dieser Schlicker wird nun mittels einer Düsenanordnung auf eine Trägerfläche aufgebracht, wobei die Düsenanordnung und die Trägerfläche relativ zueinander bewegt werden, mithin also relativ zueinander verschoben werden. Das heißt, dass durch Parallelverschiebung von Düsenanordnung und Trägerfläche letztlich beliebige Spuren mittels der Düsenanordnung auf der Trägerfläche gezogen werden können. Nach Aufbringen des Schlickers in einer ersten Schicht auf der Trägerfläche wird mittels eines Heizmittels der aufgebrachte Schlicker erwärmt, bis das Metallpulver aufschmilzt. Mit dem Aufschmelzen einher geht auch das Aufschäumen der Metallschmelze durch das vom Treibmittel freigesetzte Gas, beispielsweise Wasserstoff im Falle der Verwendung eines Metallhydrids. Anschließend kühlt die aufgeschäumte Struktur ab und verfestigt sich als Metallschaumschicht. Diese Metallschaumschicht kann nun, wie bereits beschrieben, eine nahezu beliebige, weil nur aus einer Schicht bestehend, quasi zweidimensionale Strukturen auf der Trägerfläche aufweisen.

Erfindungsgemäß werden nun die Schritte b) - d) beliebig oft wiederholt, wobei hierbei jeweils die neue Schlickerschicht auf eine bereits im Schritt zuvor aufgebrachte, aufgeschäumte und ausgehärtete Metallschaumschicht aufgebracht wird. Das heißt, dass erfindungsgemäß auf diese Weise eine filigrane und geometrisch komplexe dreidimensionale Schichtstruktur und damit ein dreidimensionales Bauteil aus Metallschaum aufgebaut werden kann, das auch in nahezu beliebiger Größe hergestellt werden kann. Hierdurch ist es also beispielsweise möglich, Trägerelemente mit einer beliebigen, innenliegenden dreidimensionalen Steg- oder Wabenstruktur oder Ähnlichem herzustellen, oder Rahmenbauteile mit entsprechenden inneren Stegstrukturen und dergleichen, also allesamt Bauteile, die einerseits eine hohe Steifigkeit respektive Festigkeit aufweisen sollen, aber auch ein möglichst geringes Gewicht. All dies wird möglich, nachdem aufgrund der relativen Verschiebebeweglichkeit von Düsenanordnung und Trägerfläche zueinander ein quasi beliebiger Verlauf der aufgetragenen Schlickerspur möglich ist und aufgrund des Schichtaufbaus auch Geometrieänderungen über die sich fortsetzende Schichtstruktur möglich sind.

Besonders zweckmäßig ist es, wenn mittels des Heizmittels nur der aufgebrachte Schlicker, nicht aber eine bereits ausgehärtete benachbarte Schicht erwärmt wird. Hierüber wird verhindert, dass eine bereits aufgeschäumte, ausgehärtete Schicht aufgrund eines zu hohen Eintrags thermischer Energie zu stark aufgeheizt wird und erneut aufschmilzt, resultierend im Verlust der lokalen Schaumstruktur.

Gemäß einer ersten Erfindungsalternative kann vorgesehen sein, dass das Heizmittel der Düsenanordnung zugeordnet ist und gemeinsam mit der Düsenanordnung relativ zur Trägerfläche bewegt wird und der aufgebrachte Schlicker mittels des Heizmittels direkt nach dem Aufbringen des Schlickers aus der Düsenanordnung erwärmt wird. Gemäß dieser Erfindungsausgestaltung bewegt sich also das Heizmittel zusammen mit der Düsenanordnung relativ zur Trägerfläche, die Düsenanordnung und das Heizmittel sind also unmittelbar benachbart zueinander, so dass es möglich ist, dass mittels des Heizmittels der Schlicker unmittelbar nach dem Aufbringen erwärmt und aufgeschäumt wird. Das heißt, dass die über die Düsenanordnung gezogene Schlickerspur unmittelbar nach dem Aufbringen über das der Düsenanordnung quasi "hinterherfahrende" Heizmittel aufgeschäumt wird.

Als Alternative ist es möglich, dass der aufgebrachte Schlicker mittels des relativ zur Trägerfläche bewegbaren Heizmittels erst nach Aufbringen der kompletten Schicht erwärmt wird. Bei dieser Ausgestaltung wird also zunächst die komplette Schlickerschicht aufgetragen, wonach in einem zweiten Schritt die aufgebrachte Schicht mit dem Heizmittel erhitzt und dadurch aufgeschäumt wird. Hierdurch kann, nachdem das Heizmittel losgelöst von der Düsenanordnung relativ zur Trägerfläche bewegt wird, verhindert werden, dass bereits aufgeschäumte Bereiche möglicherweise wieder aufgeschmolzen werden, resultierend aus einem zu nahen Heranfahren des Heizmittels.

Ist das Heizmittel der Düsenanordnung zugeordnet, wird es also mit dieser relativ zur Trägerfläche bewegt, so ist es zweckmäßig, wenn auch das Heizmittel relativ zur Düsenanordnung bewegt wird, derart, dass es bezogen auf die Relativbewegungsrichtung der Düsenanordnung zur Trägerfläche stets hinter der Düsenanordnung ist. Wie beschrieben ist es möglich, die Düsenanordnung relativ zur Trägerfläche quasi in beliebiger Richtung in Bezug auf die Aufbringebene zu verfahren. Wird mit dem Heizmittel die aufgebrachte Schlickerschicht unmittelbar nach dem Aufbringen erwärmt, also das Heizmittel mit der Düsenanordnung bewegt, so ist sicherzustellen, dass diese stets hinter der Düsenanordnung ist. Um nun entsprechende Bewegungsrichtungsänderungen diesbezüglich zu berücksichtigen, ist es möglich, dass das Heizmittel relativ zur Düsenanordnung bewegt wird, also quasi um 360° hierzu verdreht beziehungsweise um die Düsenanordnung gedreht werden kann, so dass es sich, bezogen auf die Bewegungsrichtung, stets hinter der Düsenanordnung befindet.

Wird der Schlicker erst nach Aufbringen der kompletten Schicht erwärmt, ist das Heizmittel zweckmäßigerweise längs des gleichen Relativbewegungswegs, längs dem die Düsenanordnung relativ zur Trägerfläche bewegt wurde, zu bewegen, das heißt, dass das Heizmittel dieselbe "Fahrspur" abfährt wie zuvor die Düsenanordnung.

Bezüglich der Relativbewegung von Düsenanordnung und Trägerfläche zueinander ist es entweder möglich, dass die Düsenanordnung bezüglich des positionsfesten Trägers bewegt wird, das heißt, dass die Trägerfläche ruht, während die Düsenanordnung oberhalb davon verfährt. Ist der Düsenanordnung ein Heizmittel zugeordnet, das mit ihr verfährt, so wird natürlich auch dieses Heizmittel zusammen mit der Düsenanordnung relativ zur positionsfesten Trägerfläche verfahren. Kommt ein separates Heizmittel zum Einsatz, das nach Aufbringen der gesamten Schlickerschicht diese erwärmt und quasi der vorherigen "Fahrspur" der Düsenanordnung folgt, so ist natürlich auch dieses Heizmittel relativ zur positionsfesten Trägerfläche bewegbar.

Alternativ ist es denkbar, dass die Trägerfläche relativ bezüglich der positionsfesten Düsenanordnung bewegt wird. In diesem Fall wird also die Trägerfläche unterhalb der positionsfesten Düsenanordnung verschoben. Ist das Heizmittel der Düsenanordnung zugeordnet und relativ zu dieser beweglich, also beispielsweise um 360° um sie herum fahrbar, worüber, wie gesagt, sichergestellt wird, dass das Heizmittel stets hinter der Düsenanordnung ist, so wird auch hier letztlich aktiv die Trägerfläche bezüglich des Heizmittels verschoben. Ist ein separates Heizmittel vorgesehen, so ist dieses in diesem Fall positionsfest, ähnlich wie die Düsenanordnung, und die Trägerfläche wird unterhalb des Heizmittels verschoben, um die gesamte zuvor aufgebrachte Schlickerschicht aufzuschäumen.

Als Heizmittel wird bevorzugt ein Laser verwendet, der insbesondere bei kleineren beziehungsweise schmäleren herzustellenden Schaumstrukturen zweckmäßig ist, da er eine sehr geringe Auflösung aufweist, mithin also der Eintrag der thermischen Energie lokal gesehen sehr fein und lokal niedrig aufgelöst erfolgen kann. Alternativ kann auch ein Bunsenbrenner verwendet werden, mithin also mit einer Gasflamme gearbeitet werden, wie auch eine IR-Strahlungsheizung eingesetzt werden kann.

Als Treibmittel wird zweckmäßigerweise ein Metallhydrid, beispielsweise TiH₂ verwendet. Bei entsprechender Erwärmung des Metallhydrids wird ab einer Grenztemperatur Wasserstoff freigesetzt, wobei mit zunehmender Temperaturerhöhung eine immer stärkere Freisetzung erfolgt. Die Freisetzung des Wasserstoffs beginnt bevorzugt bereits bei einer Temperatur, die unterhalb der Schmelztemperatur des Metallpulvers liegt. Wird beispielsweise TiH₂ verwendet, so beginnt die Freisetzung des Wasserstoffs bei einer Temperatur von knapp oberhalb 400°C und nimmt mit zunehmender Temperaturerhöhung immer stärker zu. Bei den üblichen verwendbaren Metallpulvern, beispielsweise Aluminiumpulver, liegt der Schmelzpunkt deutlich höher, beispielsweise liegt der Schmelzpunkt von Aluminium bei ca. 660°C, so dass mit Erreichen des Metallschmelzpunkts zwangsläufig auch eine hinreichend große Wasserstoffmenge freigesetzt wird, die sodann das Aufschäumen bewirkt.

Der Schlicker wird zweckmäßigerweise mit einem flüchtigen Lösungsmittel angemacht, wobei als Lösungsmittel zum Beispiel ein Alkohol- oder Aceton-basiertes Lösungsmittel verwendet wird, in dem das Metall- und Treibmittelpulver suspensiert wird.

Dankbar ist es des Weiteren, das Verfahren in einer sauerstoffarmen Atmosphäre durchzuführen, um eine Oxidation des verwendeten Metallpulvers respektive Metalls zu vermeiden.

Wie beschrieben können nahezu beliebige Bauteile durch diese dreidimensionale Schaumschicht-Technik hergestellt werden. Beispielsweise wird ein Bauteil einer medizinischen Untersuchungs- oder Behandlungseinrichtung, insbesondere ein C-Bogen einer Röntgeneinrichtung oder ein Rahmen einer Gantry einer Computertomographieeinrichtung, hergestellt.

Neben dem Verfahren betrifft die Erfindung ferner ein Bauteil, insbesondere eine medizinische Untersuchungs- oder Behandlungseinrichtung, vorzugsweise in Form eines C-Bogens einer Röntgeneinrichtung oder eines Rahmens einer Gantry einer Computertomographieeinrichtung, hergestellt nach dem Verfahren der vorbeschriebenen Art.

Schließlich betrifft die Erfindung eine medizinische Untersuchungs- oder Behandlungseinrichtung, umfassend ein Bauteil, wie vorstehend beschrieben.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine Prinzipdarstellung zur Erläuterung eines erfindungsgemäßen Verfahrens einer ersten Ausführungsform,
- Fig. 2: eine Prinzipdarstellung zur Erläuterung eines erfindungsgemäßen Verfahrens einer zweiten Auführungsform,
- Fig. 3: eine Prinzipdarstellung zur Erläuterung eines erfindungsgemäßen Verfahrens einer dritten Ausführungsform in einem ersten Verfahrensschritt,
- Fig. 4: eine Prinzipdarstellung entsprechend Fig. 3 zur Erläuterung eines zweiten Verfahrensschritts,
- Fig. 5: eine Prinzipdarstellung eines erfindungsgemäß hergestellten dreidimensionalen Bauteils,
- Fig. 6: eine Prinzipdarstellung zur Herstellung des Schlickers 7, und
- Fig. 7: eine medizinische Untersuchungs- oder Behandlungseinrichtung in Form einer Röntgeneinrichtung.

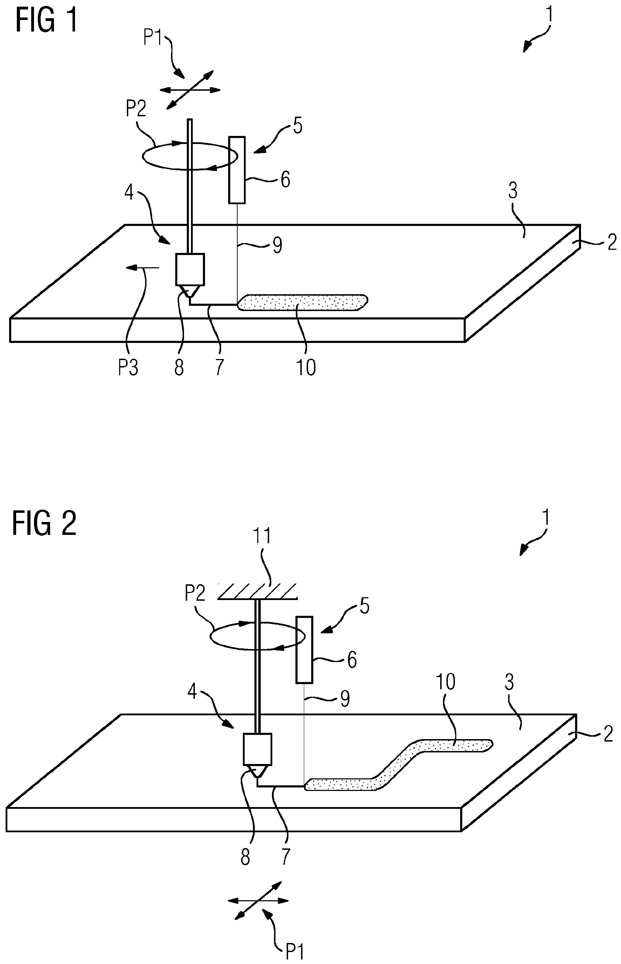

Fig. 1 zeigt eine Prinzipdarstellung einer Anordnung 1 zur Durchführung des erfindungsgemäßen Verfahrens gemäß einer ersten Erfindungsvariante. Vorgesehen ist ein Trägerbauteil 2 mit einer Trägerfläche 3 sowie eine Düsenanordnung 4, die, wie die beiden Doppelpfeile P1 darstellen, beliebig relativ zur positionsfesten Trägerfläche 3 bewegbar ist. Der Düsenanordnung 4 zugeordnet ist ein Heizmittel 5, hier in Form eines Lasers 6. Wie durch die Pfeildarstellung P2 angedeutet ist, ist das Heizmittel 5 um 360° um die Düsenanordnung 4 herum bewegbar.

Die Düsenanordnung 4 dient dazu, einen Schlicker 7 in Form einer dünner Schlickerspur auf die Trägerfläche 3 aufzubringen. Hierzu kann die Düsenanordnung 4 entweder ein Schlickerreservoir aufweisen, oder über eine Zuleitung mit einem solchen verbunden sein, um kontinuierlich den benötigten Schlicker nachführen zu können.

Der Schlicker wird im Wesentlichen aus drei Komponenten hergestellt, nämlich zum einen aus einem Metallpulver, beispielsweise Aluminiumpulver, zum anderen aus einem Treibmittel, üblicherweise ein Metallhydrid, beispielsweise TiH₂, sowie aus einem Lösungsmittel, beispielsweise auf Alkohol- oder Aceton-Basis, in dem das Metallpulver und das pulverförmige Treibmittel suspensiert werden, so dass sich eine mehr oder weniger fluide oder pastöse, in jedem Fall über die Düsenanordnung 4 ausbring- respektive aufdruckbare Masse ergibt.

Der Schlicker 7 strömt über eine Düse 8 in Form einer dünnen Schlickerschicht auf die Trägerfläche 3. In Bewegungsrichtung, wie durch den Pfeil P3 in diesem Beispiel dargestellt, gesehen hinter der Düsenanordnung 4 respektive der Düse 8, ist das Heizmittel 5 angeordnet, also der Laser 6, dessen Laserstrahl 9 auf die Schlickerschicht trifft und dies aufheizt, so dass es einerseits zum Aufschmelzen des Metallpulvers kommt, andererseits aber auch zum Freisetzen eines Schäumgases aus dem Treibmittel, üblicherweise Wasserstoff. Der Schlicker 7 schäumt, wie in Fig. 1 gezeigt, dementsprechend auf, es bildet sich eine breite Metallschaumschicht 10.

Nach dem Aufschäumen kühlt die aufgeschäumte Metallschaumschicht 10 ab, so dass demzufolge die Metallschaumschmelze erstarrt und sich der Metallschaum ausbildet.

Es liegt auf der Hand, dass infolge der Beweglichkeit der Düsenanordnung 4 in beliebiger Richtung relativ zur Trägerfläche 3 beliebige Muster respektive Spuren auf der Trägerfläche 3 abgeschieden werden können. Das Heizmittel 5 befindet sich, resultierend aus seiner Drehbarkeit um die Düsenanordnung 4, bezogen auf die Bewegungsrichtung stets hinter der Düsenanordnung 4, so dass der aus der Düse 8 ausgetretene Schlicker unmittelbar im Anschluss erhitzt und aufgeschäumt werden kann.

Nach Aufbringen einer ersten, ausgehärteten Metallschaumschicht 10 respektive nach dem Aushärten der Metallschaumschicht wird, quasi der vorherigen Spur folgend, eine weitere Schicht aufgebracht, die ebenfalls wieder aufgeschmolzen und aufgeschäumt wird und aushärtet, wonach eine dritte Schicht aufgebracht wird etc., so dass sich eine beliebige Schichtanzahl aufstapeln lässt und demzufolge ein dementsprechend großes Bauteil ausgebildet werden kann.

Fig. 2 zeigt eine Ausgestaltung ähnlich Fig. 1, wobei hier, anders als bei Fig. 1, das Trägerteil 2 und damit die Trägerfläche 3, wie durch die Doppelpfeile P1 dargestellt ist, beweglich sind, währen die Düsenanordnung 4 positionsfest ist, wie durch das Festlager 11 angedeutet ist. Lediglich das

Heizmittel 5, auch hier ein Laser 6, kann um die positionsfeste Düsenanordnung 4 gedreht werden, um sicherzustellen, dass er, bezogen auf die Relativbewegung, stets hinter der Düsenanordnung 4 ist.

Die Funktion ist genau die gleiche, mittels der Düsenanordnung 4 wird auch hier wiederum der Schlicker 7 in Form einer schmalen Schlickerspur abgegeben, wonach diese mittels des Lasers 6 aufgeschäumt wird, so dass sich die Metallschaumschicht 10 ausbildet. Wie durch die geometrisch anders geformte Metallschaumstruktur dargestellt wird, ist eine beliebige Formung möglich.

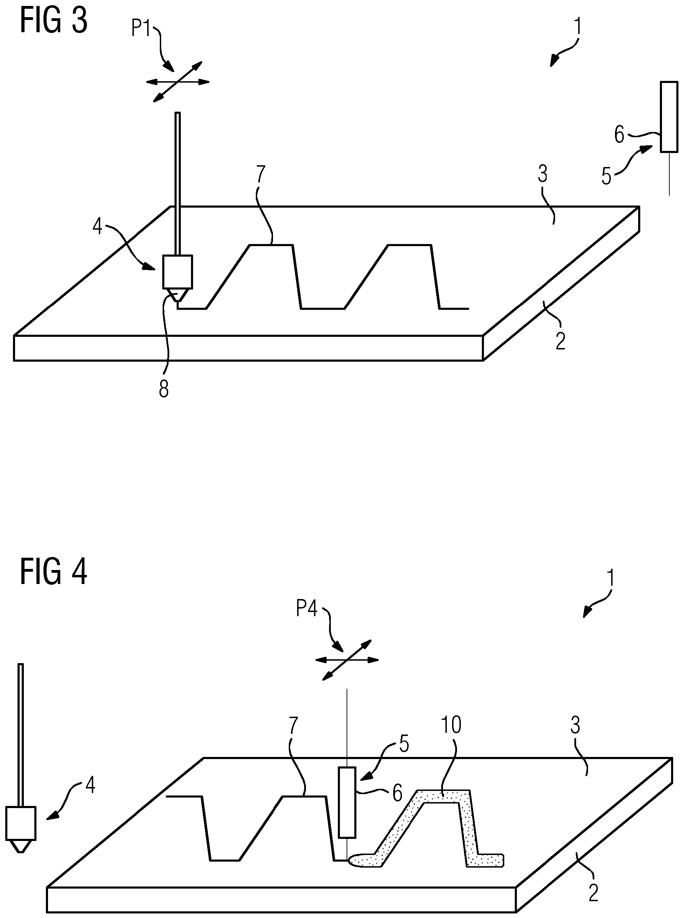

Fig. 3 zeigt eine dritte Ausgestaltung einer entsprechenden Anordnung 1, bei der das Trägerbauteil 2 mit der Trägerfläche 3 wiederum positionsfest ist und die Düsenanordnung 4, wie durch die Doppelpfeile P1 dargestellt, in jeder Richtung horizontal beweglich relativ zur Trägerfläche 3 ist. Das heißt, dass jede beliebige Richtung angefahren werden kann.

Wie Fig. 3 zeigt, wird in einem ersten Verfahrensschritt über die Düsenanordnung 4 wiederum der Schlicker 7 in Form einer schmalen Schlickerspur abgegeben, hier beispielsweise in Form einer Mäanderstruktur. Das Heizmittel 5, auch hier wiederum ein Laser 6, ist während der Schlickerabgabe in Ruhe, das heißt, dass, anders als bei den vorstehend beschriebenen Ausführungsbeispielen, bei dieser Variante zunächst der Schlicker 7 vollständig aufgebracht wird.

Fig. 4 zeigt quasi den zweiten Verfahrensschritt, nachdem über die Düsenanordnung 4 der Schlicker 7 aufgebracht wurde. Nun wird das Heizmittel 5, also der Laser 6, auf genau der gleichen Bewegungslinie bewegt, wie zuvor die Düsenanordnung 4, wie durch die Doppelpfeile P4 dargestellt ist. Das heißt, dass der Laser 6 die Schlickerschicht abfährt und zur Bildung der Metallschaumschicht 10 aufschäumt, wie Fig. 4 deutlich zeigt.

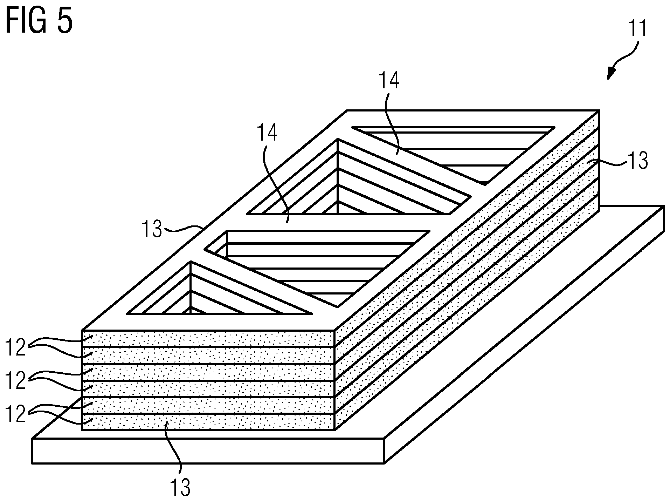

Wie bereits einleitend beschrieben, besteht ein fertig hergestelltes Bauteil 11, wie exemplarisch in Fig. 5 dargestellt ist, aus einer Vielzahl einzelner Schichten 12 aus Metallschaum, die sukzessive aufeinander aufgebracht respektive ausgebildet wurden. Ersichtlich ist es, wie in Fig. 5 angedeutet, möglich, quasi beliebige, auch filigrane Strukturen herzustellen.

Wie Fig. 5 zeigt, weist das hergestellte Bauteil insgesamt kastenförmig angeordnete Außenwände 13 auf, zwischen denen schräg verlaufende Wände 14 ausgebildet sind, also eine versteifende Innenstruktur, wobei diese Wände 14 lediglich exemplarisch dargestellt sind. Es sind beliebige andere Innenstrukturen herstellbar, beispielsweise auch in Form von schmäleren Stegen oder dergleichen, eben durch entsprechende Aufbringung des Schlickers 7 und Ausbildung der Metallschaumschichten 10 in Schichten 12 übereinander, wobei diese Schichten 12 auch geringfügig versetzt aufgebracht und erzeugt werden können, um entsprechende, schräg verlaufende Strukturen zu erzeugen.

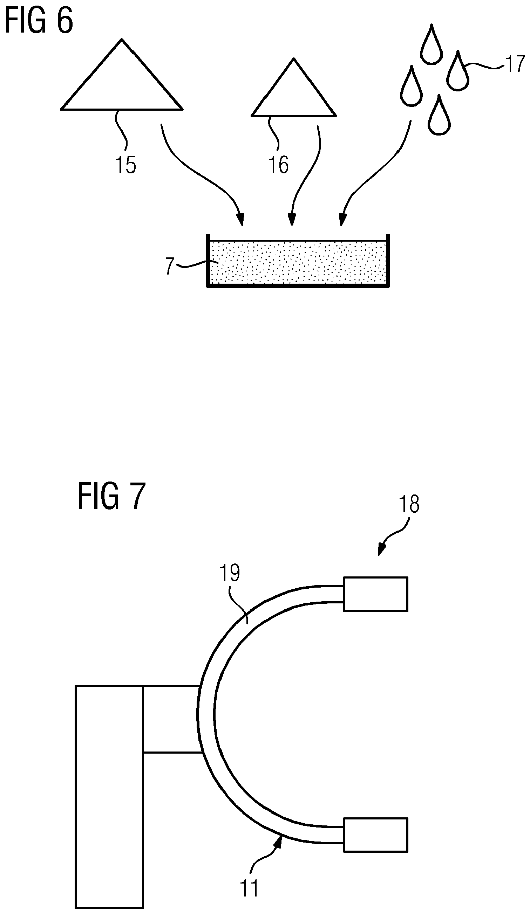

Fig. 6 zeigt eine Prinzipdarstellung zur Herstellung des Schlickers 7. Ausgangsmaterialien sind ein Metallpulver 15, beispielsweise Aluminiumpulver, ein Treibmittel 16, beispielsweise ein Metallhydrid in Form von Titandihydrid, sowie ein Lösungsmittel 17 beispielsweise auf Alkohol- oder Aceton-basis. Die Substanzen werden vermischt, um einen Schlicker 7 anzumachen, der über die Düsenanordnung 4 verarbeitet wird. Über die Düsenanordnung 4 wird er entsprechend der Düsenbreite in Form einer mehr oder weniger breiten Spur aufgezogen, wobei die Düsenbreite letztlich beliebig variieren kann.

Fig. 7 zeigt schließlich eine medizinische Untersuchungs- oder Behandlungseinrichtung 18 in Form einer Röntgeneinrichtung umfassend ein Bauteil 11 hier in Form eines C-Bogens 19, der also aus einem in dem erfindungsgemäßen Verfahren hergestellten Metallschaumkonstrukt besteht.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Herstellung eines dreidimensionalen Bauteils (11) aus einem Metallschaum, mit folgenden Schritten:
a) Verwendung eines Schlickers (7) enthaltend ein Metallpulver, ein Treibmittel und ein Lösungsmittel,
b) Aufbringen des Schlickers (7) mittels einer Düsenanordnung (4) in Form einer Schicht auf eine Trägerfläche (3), wobei die Düsenanordnung (4) und die Trägerfläche (3) relativ zueinander bewegt werden,
c) Erwärmen des aufgebrachten Schlickers (7) mittels eines Heizmittels (5) bis zum Aufschmelzen des Metallpulvers, so dass die Metallschmelze durch ein von dem Treibmittel freigesetztes Gas aufschäumt,
d) Abkühlung und Verfestigung der aufgeschäumten Schicht (10),
wobei die Schritte b) - d) beliebig oft wiederholt werden, wobei der Schlicker (7) jeweils auf eine zuvor erzeugte aufgeschäumte Schicht (10) aufgebracht wird.

2. Verfahren nach Anspruch 1, **dadurch gekennzeichnet, dass** mittels des Heizmittels (5) nur der aufgebrachte Schlicker (7), nicht aber eine bereits ausgehärtete benachbarte Schicht (10) erwärmt wird.

3. Verfahren nach Anspruch 1 oder 2, **dadurch gekennzeichnet, dass** das Heizmittel (5) der Düsenanordnung (4) zugeordnet ist und gemeinsam mit der Düsenanordnung (5) relativ zur Trägerfläche (3) bewegt wird und der aufgebrachte Schlicker (7) mittels des Heizmittels (5) direkt nach dem Aufbringen des Schlickers (7) aus der Düsenanordnung (4) erwärmt wird, oder dass der aufgebrachte Schlicker (7) mittels des relativ zur Trägerfläche (3) bewegbaren Heizmittels (5) erst nach Aufbringen der kompletten Schicht erwärmt wird.

4. Verfahren nach Anspruch 3, **dadurch gekennzeichnet, dass** das der Düsenanordnung (4) zugeordnete Heizmittel (5) relativ zur Düsenanordnung (4) bewegt wird, derart, dass es bezogen auf die Relativbewegungsrichtung der Düsenanordnung (4) zur Trägerfläche (3) stets hinter der Düsenanordnung (4) ist.

5. Verfahren nach Anspruch 3, **dadurch gekennzeichnet, dass** das den Schlicker (7) erst nach Aufbringen der kompletten Schicht erwärmende Heizmittel (5) längs desgleichen Relativbewegungswegs, längs dem die Düsenanordnung (4) relativ zur Trägerfläche (3) bewegt wurde, bewegt wird.

6. Verfahren nach einem der vorangehenden Ansprüche, **dadurch gekennzeichnet, dass** die Düsenanordnung (4) bezüglich der positionsfesten Trägerfläche (3) bewegt wird, oder dass die Trägerfläche (3) relativ bezüglich der positionsfesten Düsenanordnung (4) bewegt wird.

7. Verfahren nach einem der vorangehenden Ansprüche, **dadurch gekennzeichnet, dass** das Heizmittel (5) ein Laser (6), ein Bunsenbrenner oder eine IR-Strahlungsheizung ist.

8. Verfahren nach einem der vorangehenden Ansprüche, **dadurch gekennzeichnet, dass** als Treibmittel ein Metallhydrid, insbesondere TiH₂ verwendet wird.

9. Verfahren nach einem der vorangehenden Ansprüche, **dadurch gekennzeichnet, dass** als Metallpulver Aluminiumpulver verwendet wird.

10. Verfahren nach einem der vorangehenden Ansprüche, **dadurch gekennzeichnet, dass** als Lösungsmittel ein flüchtiges Lösungsmittel, insbesondere ein Alkohol- oder Aceton-basiertes Lösungsmittel verwendet wird.

11. Verfahren nach einem der vorangehenden Ansprüche, **dadurch gekennzeichnet, dass** es in einer sauerstoffarmen Atmosphäre durchgeführt wird.

12. Verfahren nach einem der vorangehenden Ansprüche, **dadurch gekennzeichnet, dass** ein Bauteil (11) einer medizinischen Untersuchungs- oder Behandlungseinrichtung (18), insbesondere ein C-Bogen (19) einer Röntgeneinrichtung oder ein Rahmen einer Gantry einer Computertomographieeinrichtung, hergestellt wird.

13. Bauteil (11), insbesondere einer medizinischen Untersuchungs- oder Behandlungseinrichtung (18), vorzugsweise in Form eines C-Bogens (19) einer Röntgeneinrichtung oder eines Rahmen einer Gantry einer Computertomographieeinrichtung, hergestellt nach dem Verfahren nach einem der vorangehenden Ansprüche.

14. Medizinische Untersuchungs- oder Behandlungseinrichtung, umfassend ein Bauteil (11) nach Anspruch 13.
